# EUROPEAN PATENT SPECIFICATION

(11) **EP 1 997 522 B1**
(45) Date of publication and mention of the grant of the patent: **13.05.2015**
(21) Application number: 07739014.4
(22) Date of filing: 19.03.2007
(51) Int. Cl.: A61L 27/04, A61L 27/30, A61L 27/58, A61L 31/08, A61L 31/14, A61L 31/02

(54) **METHOD OF CONTROLLING DEGRADATION TIME OF A BIODEGRADABLE DEVICE**
VERFAHREN ZUR KONTROLLE DER ABBAUSZEIT EINER BIOLOGISCH ABBAUBAREN VORRICHTUNG
PROCÉDÉ DE CONTRÔLE DU TEMPS DE DÉGRADATION D'UN DISPOSITIF BIODEGRADABLE

(30) Priority: 20.03.2006 JP 2006077776
(43) Date of publication of application: 03.12.2008
(73) Proprietor: National Institute for Materials Science, Tsukuba-shi, Ibaraki 305-0047 (JP)
(72) Inventor: HIROMOTO, Sachiko, Tsukuba-shi, Ibaraki 3050047 (JP); YAMAMOTO, Akiko, Tsukuba-shi, Ibaraki 3050047 (JP); MARUYAMA, Norio, Tsukuba-shi, Ibaraki 3050047 (JP); MUKAI, Toshiji, Tsukuba-shi, Ibaraki 3050047 (JP); SOMEKAWA, Hidetoshi, Tsukuba-shi, Ibaraki 3050047 (JP)
(74) Representative: Calamita, Roberto
(86) International application number: PCT/JP2007/055571
(87) International publication number: WO 2007/108450

(56) References cited:
- JP-A- 2001 181 772
- JP-A- 2002 028 229
- JP-A- 2002 511 782
- JP-A- 2003 190 272
- JP-A- 2003 500 159
- JP-A- 2004 323 908
- JP-A- 2005 021 420
- JP-A- 2005 518 830
- US-A1- 2006 052 824

## Description

### Technical Field

The present invention relates to a method of controlling the degradation time of biodegradable magnesium based metallic material for medical use. More particularly, the invention relates to providing a biodegradable magnesium based metallic material for medical use which is capable of exhibiting desired mechanical properties at an early stage of implantation without changing the mechanical properties such as strength and ductility inherent to magnesium or its alloy and also controlling the retention time of the mechanical property to be short or long in a desired manner and a method for producing the same.

### Background Art

A metallic device for medical use which has been generally and conventionally used remains in the body unless it is removed from the body by a surgical operation or the like after being implanted in the body. Depending on its intended purpose, it is desired that such a device should retain the strength during the period for which surrounding tissue is healed and should be degraded and disappeared after healing without requiring a surgical operation. A magnesium based metallic material is a material with a low toxicity for the living body, and corrodes at a very high rate in an aqueous solution having a near-neutral pH in which chloride ions are present such as a body fluid and is degraded and disappeared. Accordingly, it has been expected that such a magnesium based metallic material is used as a biodegradable metallic material for medical use which is gradually degraded and absorbed after it is implanted in the body, and its development has been advanced (see, for example, Patent documents 1 and 2).

However, the retention time of the strength required for the device varies in a very wide range from long to short depending on the type of device or the condition of an affected area. For example, it is desired that a device for treatment of a blood vessel such as a stent should retain the strength for a period of 5 days to 6 months required for healing of a narrowed area of the blood vessel, and after the blood vessel is healing, the degradation of the entire device should be substantially completed for a period of 1 week to 12 weeks. This is because when the stent remains after the blood vessel wall is healed, restenosis of a blood vessel is caused by excessive proliferation of vascular endothelial cells due to the mechanical and chemical stimulation which is continuously given to the blood vessel wall by the stent, therefore, it is very important to eliminate the stent after the blood vessel is healed. On the other hand, as a fracture fixation device, it is desired that the device should support a load for a period of 3 months to 1 year until bone fracture is healed, and thereafter, the degradation of the entire device should be substantially completed for a period of 8 months to 5 years. In this way, a load is gradually applied to the healed bone accompanying the degradation and disappear of the device after bone fracture is healed, therefore, it is possible to suppress stress shielding, which means that the device supports the load instead of bone. This leads to suppression of refracture which is caused by bone resorption (bone is dissolved and thinned) due to stress shielding, and it is not necessary to perform a surgical operation to remove the device after bone fracture is healed, and thus, the burden of patients can be reduced. As described above, the retention time of a required strength varies in a wide range depending on the device, and a long period of time of several months or more is required in some cases. Further, it is considered that the progress of degradation can be preferably controlled depending on the period for which retention of strength is required and the period for which the device is degraded thereafter.

For example, the biodegradable magnesium based metallic material proposed in Patent document 1 is designed such that the period of degradation is controlled depending on the size of device. However, the degradation begins immediately after it is implanted, and further, it is practically impossible to suitably use the biodegradable magnesium based metallic material as a device which requires long-term strength retention in the body in which a desired size of device or the space to be implanted is limited.

Further, the biodegradable magnesium based metallic material proposed by the present inventors in Patent document 2 is designed such that the strength and ductility balance of the material and the degradation rate in the body are controlled to a desired value by controlling the composition or structure of the material itself. For example, as the grain size of magnesium is made finer, the degradation can be accelerated, and by making the grain size large or varying the kind of element to be added and controlling concentration of the element added, the degradation rate can be decreased. However, in the case where the grain size is made large, it becomes difficult to perform fine adjustment of the degradation rate, and it is relatively difficult to decrease the degradation rate precisely. That is, because the degradation begins immediately after it is implanted, it is difficult to control both of the retention of the strength at an early stage of implantation and the degradation rate which achieves long-term degradation in various ways.

On the other hand, a technique in which the corrosion resistance of pure magnesium for biomedical use is improved by thermal oxidation thereof in an oxidation atmosphere and the mechanical strength inherent to magnesium is utilized has also been proposed (Patent document 3). However, the thermal treatment at a high temperature in Patent document 3 changes the microstructure of the magnesium based metallic material serving as a substrate which leads to deterioration of strength or corrosion resistance, therefore, it has a problem that the magnesium based metallic material which can be subjected to thermal oxidation is limited. Further, the oxide film formed on the surface of the magnesium based metallic material by thermal oxidation cannot sufficiently suppress the degradation of the magnesium based metallic material for a long period of time after it is implanted in the body.

As described above, the current situation is that in the conventional biodegradable magnesium based metallic materials for medical use, it is difficult to control the degradation for a long period of time as well as to exhibit a desired mechanical property at an early stage of implantation without changing the mechanical properties inherent to magnesium or its alloy such as strength and ductility and also it is difficult to control the retention time of the mechanical property to be short or long in a desired manner.
Patent document 1: JP-A-2004-160236
Patent document 2: Japanese Patent Application No. 2005-331841
Patent document 3: JP-A-2002-28229

US2006052824 discloses a method of forming a magnesium oxide coating, by anodic oxidation, on a medical device comprising magnesium or a magnesium alloy. Said coating being arranged in order to provide a preferential corrosion zone.

### Disclosure of Invention

### Problems that the Invention is to Solve

Accordingly, the present invention has been made in view of the above circumstances and has an objective to solve the problems of the prior art and to provide a biodegradable magnesium based metallic material for medical use which is capable of exhibiting a desired mechanical property at an early stage of implantation without changing the mechanical properties inherent to magnesium or its alloy such as strength and ductility and also controlling the retention time of the mechanical property to be short or long in a desired manner and a method for producing the same.

### Means for Solving the Problems

In a first aspect, the present invention provides a method of controlling a degradation time of a biodegradable device for medical use, which is a method of controlling a degradation time in vivo of a biodegradable device for medical use containing a biodegradable magnesium material or magnesium alloy material for medical use which is degraded and absorbed in vivo as a substrate, characterized by controlling the degradation time in vivo by forming a film containing magnesium oxide and/or magnesium hydroxide by anodic oxidation on the surface of the substrate and controlling morphology of the film thereby controlling the porosity or pore size of the film, wherein the anodic oxidation is effected by applying charge using magnesium or a magnesium alloy as an anode in an electrolyte containing one or more compositions selected from the group consisting of a salt or a hydroxide of sodium, potassium, aluminium or calcium, a salt of phosphoric acid, silicic acid, aluminic acid, boric acid, oxalic acid, acetic acid or tartaric acid, a fluoride and ethylene glycol.

In the biodegradable magnesium based metallic material for medical use, average grain size thereof may be not more than one-fourth of a minimal part of the material.

The biodegradable magnesium based metallic material for medical use may contain 93.5 atomic % or more of magnesium as a main composition and further contains a secondary composition, and that the concentration of the secondary composition unevenly distributed at a grain boundary is 1.2 times or more the average concentration thereof within the grain.

The third biodegradable magnesium based metallic material for medical use may contain, as a secondary composition, any one element selected from 0.03 atomic % or less of Ce, 0.03 atomic % or less of Pr, 0.033 atomic % or less of Au, 0.043 atomic % or less of Ir, 0.047 atomic % or less of La, 0.067 atomic % or less of Pd, 0.17 atomic % or less of Th, 0.21 atomic % or less of Nd, 0.3 atomic % or less of Ca, 0.3 atomic % or less of Mn, 0.35 atomic % or less of Zr, 0.37 atomic % or less of Bi, 0.4 atomic % or less of Yb, 0.47 atomic % or less of Rb, 0.64 atomic % or less of Co, 0.8 atomic % or less of Zn, 0.8 atomic % or less of Pu, 1.0 atomic % or less of Ga, 1.3 atomic % or less of Y, 1.3 atomic % or less of Ag, 1.5 atomic % or less of Gd, 1.6 atomic % or less of Dy, 1.8 atomic % or less of Ho, 2.1 atomic % or less of Tm, 2.4 atomic % or less of Er, 3.0 atomic % or less of Lu, 3.9 atomic % or less of Al, 5.0 atomic % or less of Sc, 5.7 atomic % or less of Li and 6.5 atomic % or less of in, and the remainder are inevitable impurities.

In the biodegradable magnesium based metallic materials for medical use, it is characterized in that the film is porous.

In the method of the invention, the time, voltage and current of electric charge may be controlled to obtain the desired morphology of the film.

### Advantage of the invention

The biodegradable magnesium based metallic material for medical use resulting from the method of the invention is capable of exhibiting a desired mechanical property at an early stage of implantation in the body without changing the mechanical properties inherent to magnesium or its alloy such as strength and ductility by forming a film, which contains magnesium oxide and magnesium hydroxide, on the surface of magnesium or its alloy by anodic oxidation thereby suppressing deterioration of the mechanical strength of magnesium or its alloy.

Further, the morphology such as structure, thickness or composition of the film of the biodegradable magnesium based metallic material for medical use resulting from the method of the invention can be changed in various ways according to the conditions of anodic oxidation, and the protectiveness of the film in vivo, that is, a period until the film is broken down and the degradation of the substrate of the magnesium based metallic material begins can be controlled to be short or long in a desired manner, in other words, the retention time of the mechanical property can be controlled to be short or long in a desired manner.

In addition to the above effects, the biodegradable magnesium based metallic material for medical use resulting from the method of the invention also exhibits effects as described below.

On the surface of the film of the biodegradable magnesium based metallic material for medical use of the invention, calcium phosphate is precipitated, and its precipitation amount or structure is changed depending on the implanted site in the body. Therefore, the bone formation is accelerated on the surface of the film of the magnesium based metallic material implanted around the bone tissue, and connectivity between the material and bone is increased. On the other hand, the surface of the film of the magnesium based metallic material on which calcium phosphate is precipitated has a high compatibility with soft tissue, therefore, in the case where the magnesium based metallic material is implanted in the blood vessel, it shows a high compatibility with soft tissue because calcium phosphate is precipitated on the surface of the film of the magnesium based metallic material at an early stage. Accordingly, a biodegradable magnesium based metallic material for medical use with improved biocompatibility and connectivity is provided. Further, such a material can be expected to serve as a device for regenerative medicine which is replaced with regenerated bone accompanying degradation and absorption of magnesium like an artificial bone, a skull plate or the like to be implanted in a bone defect area.

Further, the film can be made porous, and a drug or a protein is loaded in the pores of the film, whereby a biodegradable magnesium based metallic material for medical use enabling sustained release in vivo is provided. Further, by controlling the pore size, it becomes possible to control the type of drug or protein to be loaded or the sustained release rate thereof.

### Brief Description of Drawings

[Fig. 1] Photographs showing scanning electron microscopic (SEM) images of as-polished surface of a binary magnesium alloy. (a2) is a photograph of (a1) with high magnification.
[Fig. 2] Photographs showing scanning electron microscopic (SEM) images of the surface of an anodic oxide film formed by anodic oxidation at 2 V on a binary magnesium alloy. (b2) is a photograph of (b1) with high magnification.
[Fig. 3] Photographs showing scanning electron microscopic (SEM) images of the surface of an anodic oxide film formed by anodic oxidation at 7 V on a binary magnesium alloy. (c2) is a photograph of (c1) with high magnification.
[Fig. 4] Photographs showing scanning electron microscopic (SEM) images of the surface of an anodic oxide film formed by anodic oxidation at 10 V on a binary magnesium alloy. (d2) is a photograph of (d1) with high magnification.
[Fig. 5] Photographs showing scanning electron microscopic (SEM) images of the surface of an anodic oxide film formed by anodic oxidation at 20 V on a binary magnesium alloy. (e2) is a photograph of (e1) with high magnification.
[Fig. 6] Photographs showing scanning electron microscopic (SEM) images of the surface of an anodic oxide film formed by anodic oxidation at 100 V on a binary magnesium alloy. (f2) is a photograph of (f1) with high magnification.
[Fig. 7] Photographs showing scanning electron microscopic (SEM) images of the surface of an anodic oxide film formed by anodic oxidation at 200 V on a binary magnesium alloy. (g2) is a photograph of (g1) with high magnification.
[Fig. 8] A graph showing the thickness of an oxide film on the as-polished surface and the surface anodically oxidized of a binary magnesium alloy.
[Fig. 9] Graphs showing the X-ray photoelectron spectroscopy (XPS) spectra of the as-polished surface and the surface anodically oxidized of a binary magnesium alloy. (a) shows Mg 2p electron spectra; (b) shows Mg KLL Auger electron spectra; and (c) shows Y 3d electron spectra.
[Fig. 10] A graph showing a relationship between a film breakdown potential and an anodic oxidation voltage in an artificial body fluid for a magnesium based metallic material.
[Fig. 11] Graphs showing a transient curve of the immersion potential in an artificial body fluid for a magnesium alloy (AZ31 extruded material): (a) as-polished; and anodically oxidized (b) at 7 V; (c) at 100 V; and (d) at 200 V.
[Fig. 12] Photographs showing scanning electron microscopic (SEM) images of the surface of a binary magnesium alloy subjected to thermal oxidation. (a2) is a photograph of (a1) with high magnification; and (a3) is a photograph of (a2) with high magnification.
[Fig. 13] A graph showing a film breakdown potential in an artificial body fluid for a binary magnesium alloy subjected to thermal oxidation or anodic oxidation.
[Fig. 14] Photographs showing stereo microscopic images of the surface of binary magnesium alloys subjected to (a) thermal oxidation and (b) anodic oxidation at 7 V immersed in an artificial body fluid for 2 weeks.
[Fig. 15] A schematic illustration showing a method of polarization test in an artificial body fluid.
[Fig. 16] A schematic illustration showing a method of an immersion test in an artificial body fluid.

### Description of Reference Numerals and Signs

(1) Sample
(2) Fixing member
(3) Stainless steel plate
(4) Reference electrode
(5) Counter electrode
(6) Vessel
(7) Artificial body fluid
(8) Electric wire
(9) Potentiostat
(10) Digital X-Y recorder

### Best Mode for Carrying Out the Invention

The present invention has characteristics as described above, and an embodiment thereof will be described hereunder.

The biodegradable magnesium based metallic material for medical use resulting from the method of the invention is characterized by comprising a film, which mainly contains magnesium oxide and magnesium hydroxide and is formed on the surface of magnesium or a magnesium alloy by anodic oxidation.

This biodegradable magnesium based metallic material for medical use can be used as a biodegradable material for medical use which is implanted in the body, and thereafter is gradually degraded and absorbed in the body, and its morphology such as shape or size can be arbitrarily determined according to the intended purpose.

It is understood that the biodegradable magnesium based metallic material for medical use resulting from this method of the invention has a film formed on the surface of a magnesium based metallic material serving as a substrate by anodic oxidation, and as magnesium or a magnesium alloy serving as a substrate, a biodegradable magnesium based metallic material which has already been proposed by the present inventors and in which the degradation rate after it is implanted in the body is controlled while the strength and ductility balance is maintained high (see JP-A-2005-331841) can be used. Specifically, it is a magnesium based metallic material which comprises magnesium with an impurity concentration of 0.05 atomic % or less and in which an average grain size thereof is controlled to be not more than one-fourth of a minimal part of the material; or a magnesium based metallic material which contains 93.5 atomic % or more of magnesium as a main composition, as a secondary composition, any one element selected from 0.03 atomic % or less of Ce, 0.03 atomic % or less of Pr, 0.033 atomic % or less of Au, 0.043 atomic % or less of Ir, 0.047 atomic % or less of La, 0.067 atomic % or less of Pd, 0.17 atomic % or less of Th, 0.21 atomic % or less of Nd, 0.3 atomic % or less of Ca, 0.3 atomic % or less of Mn, 0.35 atomic % or less of Zr, 0.37 atomic % or less of Bi, 0.4 atomic % or less of Yb, 0.47 atomic % or less of Rb, 0.64 atomic % or less of Co, 0.8 atomic % or less of Zn, 0.8 atomic % or less of Pu, 1.0 atomic % or less of Ga, 1.3 atomic % or less of Y, 1.3 atomic % or less of Ag, 1.5 atomic % or less of Gd, 1.6 atomic % or less of Dy, 1.8 atomic % or less of Ho, 2.1 atomic % or less of Tm, 2.4 atomic % or less of Er, 3.0 atomic % or less of Lu, 3.9 atomic % or less of Al, 5.0 atomic % or less of Sc, 5.7 atomic % or less of Li and 6.5 atomic % or less of In, and as the remainder, inevitable impurities.

Further, it is a magnesium based metallic material or the like in which the concentration of the secondary composition unevenly distributed at a grain boundary is controlled to be 1.2 times or more the average concentration thereof within the grain. This magnesium based metallic material is capable of controlling the degradation rate in vivo while achieving a desired mechanical property such as strength, work hardenability or ductility required for individual devices by controlling the composition of the material and the grain size in various ways. That is, by using such a magnesium based metallic material as a substrate, the degradation rate of the substrate of the biodegradable magnesium based metallic material for medical use of the invention can be controlled in a desired manner.

Further, the distinctive film of this biodegradable magnesium based metallic material for medical use functions as a protective film for magnesium or a magnesium alloy serving as a substrate, and is capable of keeping a period from immediately after the magnesium based metallic material is implanted into the body until it begins to be degraded such that the period fits the intended purpose and surely retaining the strength inherent to the substrate during the period. It was found by the studies made by the present inventors that as for such a film, a film, which contains magnesium oxide and magnesium hydroxide and is formed by anodic oxidation is most preferred as the biodegradable material for medical use.

The thickness of the film can be arbitrarily determined according to the period until the magnesium or a magnesium alloy serving as the substrate begins to be degraded.

Further, this film can be characterized In that it contains magnesium oxide and magnesium hydroxide because it is formed by anodic oxidation of a magnesium based metallic material as described above. The precipitation amount or structure of calcium phosphate is changed depending on the ratio of the magnesium oxide or magnesium hydroxide or the structure thereof. Such calcium phosphate accelerates the bone formation and improves connectivity between the material and bone and also it has good compatibility with vascular endothelial cells. Because of such characteristics, the biodegradable magnesium based metallic material for medical use resulting from the method of the invention is achieved such that it has a function to accelerate precipitation of calcium phosphate from the body fluid and has an extremely high biocompatibility. That is, when a biodegradable device for medical use made of this biodegradable magnesium based metallic material for medical use is implanted in the body, the surface of the device moderately connects to surrounding tissue, the device has good compatibility with surrounding tissue cells, and the biocompatibility of the surface is high, therefore, it is expected, for example, that the biodegradable device for medical use will begin to heal the surrounding tissue at an initial stage of implantation and complete the healing at an early stage without causing, for example, thrombus formation.

Further, because this film is formed through anodic oxidation, the structure, thickness, composition or the like of the film can be changed in various ways, and thus, it is possible to adjust the protectiveness or biocompatibility of the film. For example, depending on the conditions of anodic oxidation or the conditions such as the type and concentration of an electrolyte to be used, the composition, morphology and the like of the film can be controlled in various ways. Specifically, for example, the biodegradable magnesium based metallic material for medical use resulting from the method of the invention can be designed such that a constituent element and compound derived from an electrolyte or the like can be incorporated in the film in addition to magnesium oxide and magnesium hydroxide. Further, the surface of the film can be made smooth, or it can be made porous and the pore size can also be changed. For example, specifically, the biodegradable magnesium based metallic material for medical use of the invention can be designed to have pores with a pore size of 1 µm or less on the film, although it is not limited thereto. Further, for example, it becomes possible to control the ability to precipitate calcium phosphate or the structure of crystal to be precipitated.

Recently, a treatment in which healing of lesion is accelerated by supplying a drug from the surface of a biomaterial has been performed, and the surface morphology such as pores of the biodegradable magnesium based metallic material for medical use resulting from the method of the invention can be utilized for the purpose of loading a drug and releasing a drug in a sustained manner. In this way, the surface of the biodegradable magnesium based metallic material for medical use of the invention of this application can have a function to release a drug for accelerating the healing of surrounding tissue in addition to high biocompatibility. For example, specifically, in the case where a fracture fixation device is made of the biodegradable magnesium based metallic material for medical use of the invention, a protein which is a bone growth factor or the like is loaded in the pores of the film in advance and after the device is implanted in the body, the protein is released in a sustained manner from the surface of the device, whereby a treatment in which bone formation is accelerated, whereby healing of bone fracture is accelerated can be proposed. Further, in the case of a stent, it is possible to perform a treatment of preventing abnormal growth of vascular endothelial cells by supplying a drug from the surface of a stent in order to prevent restenosis caused by abnormal growth of vascular endothelial cells due to the continuous mechanical stimulation given to the blood vessel wall by the stent. Further, the strength and elasticity of the blood vessel wall with a lesion are lower than those of the normal blood vessel wall, and they cannot return to the strength and elasticity of the normal blood vessel wall only by expanding the blood vessel wall with the stent. Therefore, a treatment in which a drug for accelerating healing of the blood vessel wall is released in a sustained manner from the surface of the stent can also be achieved. Further, for example, by implanting a device (a drug eluting device for medical use) loading a drug in the bone of a patient with osteoporosis and the drug is released in a sustained manner from the device, and a treatment in which an increase in the bone weight is accelerated can be achieved. Such a porous surface of the biodegradable magnesium based metallic material for medical use of the invention can be utilized as a surface for sustained release of a drug in which a drug is loaded in the pores and is released in a sustained manner in the body. Moreover, it is also considered that by controlling the pore size or porosity, an adjustment function such that any of various types of drugs is loaded or a drug is released in a sustained manner at a rate suitable for a lesion may be imparted.

With regard to the biodegradable magnesium based metallic material for medical use of the invention as described above, control of the formation of the film by anodic oxidation and its morphology can be performed regardless of the composition or structure of a magnesium based metallic material serving as the substrate, and further, it does not affect the microstructure of the magnesium based metallic material. Therefore, magnesium or a magnesium alloy serving as the substrate can maintain a inherent strength and ductility balance, degradation property and the like without causing damage of its composition or structure.

The above-mentioned biodegradable magnesium based metallic material for medical use of the invention can be produced by a method provided by the invention. That is, the method for producing the biodegradable magnesium based metallic material for medical use of the invention comprises forming a film, which mainly contains magnesium oxide and magnesium hydroxide, on the surface of magnesium or a magnesium alloy by anodic oxidation with electrically charging using the magnesium or magnesium alloy as an anode in an electrolyte.

As the magnesium or magnesium alloy serving as the substrate, the magnesium based metallic material (see Japanese Patent Application No. 2005-331841) which has already been proposed by the present inventors can be used as described above. The morphology of the substrate can be determined such that the substrate has a size and a shape suitable for achieving the intended purpose. Such magnesium or a magnesium alloy serving as the substrate has the composition as described above, and an average grain size thereof is controlled to be not more than one-fourth of a minimal part of the material. The control of the average grain size can be achieved by, for example, utilizing structural control by a processing. Specifically, the control of grain size can be achieved by performing a large deformation process, for example, an extrusion and rolling process or the like, at a temperature not lower than the temperature at which recrystallization of the material occurs. For example, more specifically, although it depends on the composition of a mother alloy, a process in which after a homogenization treatment at a temperature in a range from about 450 to 550°C for about 1.5 to 8 hours is performed, quenching is performed to freeze the resulting homogeneously dispersed structure, and then warm deformation at a temperature in a range from about 80 to 350°C is applied, or the like can be mentioned as an example. The control of the average grain size is not limited to this extrusion and rolling process, however, in the case where the control is achieved by such an extrusion and rolling process, a severe process at a temperature not lower than the recrystallization temperature as described above is indispensable. Further, an extrusion ratio (cross-sectional area ratio) in this case is, for example, about 16 to 100, and therefore, the extrusion process is performed such that it becomes a more severe process than a normal extrusion process, which is mentioned as a preferred example.

In the case where the substrate is a magnesium alloy, by controlling the solid solution state of the secondary composition in Mg and the uneven distribution state of the secondary composition in a grain boundary, the strength and ductility balance and degradation rate are controlled to a desired value. The control of the solid solution state and uneven distribution state in a grain boundary of the second composition can be achieved by selecting the composition and also utilizing structural control by a processing. Specifically, the control of the solid solution state and uneven distribution state in a grain boundary of the second composition can be achieved by adjustment of the concentration of the second composition and grain size.

With regard to the electrolyte or atmosphere, in order to prevent an element exhibiting toxicity to the living body from being incorporated in the film to be formed, it is preferred that the electrolyte or atmosphere does not contain an element exhibiting toxicity to the body such as Mn or Cr. As such an electrolyte, for example, a known anodic oxidation solution can be used. Specific examples thereof can include a solution obtained by adding a phosphate, sodium aluminate, a fluoride or the like to a strong alkaline aqueous solution of such as sodium hydroxide, potassium hydroxide or ammonium acetate as a base. Such an electrolyte is useful as a solution which does not allow an element exhibiting toxicity to the body to remain in the film. As described above, in the invention, it can be considered that as a composition of the electrolyte for example, one or more compositions selected from the group consisting of a salt or a hydroxide of sodium, potassium, aluminum or calcium, a salt of phosphoric acid, silicic acid, aluminic acid, boric acid, oxalic acid, acetic acid or tartaric acid, a fluoride and ethylene glycol are contained. More specifically, for example, compositions such as sodium phosphate, sodium hydrogen phosphate, potassium fluoride, sodium fluoride, aluminum fluoride, sodium silicate, sodium borate, sodium aluminate, sodium oxalate, aluminum hydroxide, ammonium tartrate and ethylene glycol can be mentioned as the examples. In particular, an electrolyte obtained by adding a fluoride or the like can be used in the case where the film is made porous, or for the purpose of facilitating the control of porosity or pore size of the film. Further, for example, in the case of a solution containing Al ions, Al can be incorporated in the film as an oxide or a composite oxide with Mg. As described above, for example, by changing the condition such as the composition or concentration of the electrolyte, an element in the solution can be incorporated in the film, or the morphology such as porosity or pore size of the film can be changed.

With regard to the condition for anodic oxidation, the voltage, current and treatment time can be changed according to the desired protectiveness or biocompatibility and morphology of the resulting film. In general, as the treatment time is set longer, the thickness of the film is increased. Further, by controlling the voltage, the thickness or morphology of the film can be changed, therefore, it becomes possible to control the degradation of the substrate at an early stage of implantation thereof into the body over a desired period of time. Further, by controlling the voltage and current, the surface morphology of the film can be controlled. It is not necessarily appropriate to suggest because the surface morphology of the film varies depending on the size or shape of the substrate, the composition of the electrolyte or the like, however, for example, by setting the voltage to a low voltage of around 5 V and a high voltage not lower than the dielectric breakdown voltage of the film, the film can be made porous. Further, the pore size and porosity can be controlled. Further, depending on the voltage, a constituent element of the substrate or electrolyte can be incorporated in the film, and the composition of the film can be changed.

In the invention as described above, the surface of a magnesium based metallic material is treated by anodic oxidation. As the surface treatment technique, other than an anodic oxidation technique, a technique of chemical conversion treatment, electroplating, enameling, ion plating, or sputtering is generally employed, and a hydrothermal treatment or a thermal oxidation treatment in an oxidation atmosphere can also be employed. However, in a chemical conversion treatment specified in JIS standard, almost all treatment solutions contain sodium bichromate, and the treatment is intended to form a chromate film. Recently, a chromium-free chemical conversion treatment in which hexavalent chromium is not used has been performed, however, a treatment solution containing manganese instead of hexavalent chromium is used in many cases. Hexavalent chromium and manganese have high toxicity to the body, and the possibility that hexavalent chromium or manganese remains on the surface subjected to a chemical conversion treatment cannot be ignored, therefore, the current chemical conversion treatment is determined to be not suitable as a surface treatment method for the biodegradable magnesium based metallic material for medical use.

Further, any of the techniques of electroplating, enameling, ion plating and sputtering is a method of coating the surface of a material with a metal or a metal oxide of a composition different from that of an underlying material, and the control of the structure, thickness or composition of the film is relatively simple. However, for example, in the case where a nobler metal than magnesium is contained in the film such as plating, when magnesium in the underlying material is exposed to the surface in a state in which a plating layer remains in the body, a galvanic cell is formed there, and local corrosion of magnesium is greatly accelerated. Such local corrosion leads to break of a part of a device or rapid breakdown thereof, and there is a problem that a risk factor (for example, a piece of such a device may be released in the blood) cannot be eliminated.

Further, as the thermal oxidation treatment of a magnesium based metallic material for biomedical use in an oxidation atmosphere, a treatment performed at a heating temperature of 400 to 600°C for 3 to 100 hours has been proposed ( JP-A-2002-25229 ). According to this method, some types of magnesium based metallic materials may cause coarsening of grains, which leads to deterioration of strength or ductility, therefore, it is not preferred to produce a device which requires a strength for a long period of time.

On the other hand, an autoclave treatment which is one type of hydrothermal treatment is performed generally under a condition of 120 to 121°C for about 15 to 30 minutes as one of the methods of sterilization of a biomaterial. Since it is unlikely to cause coarsening of grains of pure magnesium or a magnesium alloy under the above-mentioned condition, it is considered that as well as the anodic oxidation of this application, the autoclave treatment can be a method effective in formation of the film and modification for the biodegradable magnesium based metallic material for medical use.

The biodegradable magnesium based metallic material for medical use resulting from the method of the invention as described above is a biodegradable magnesium based metallic material for medical use in which the degradation thereof at an early stage of implantation in the body is suppressed and connectivity to surrounding tissue such as bone, that is, the biocompatibility and the connectivity are improved, and can be used as a biodegradable device for various medical uses. For example, as specific examples, the biodegradable magnesium based metallic material for medical use is effective in the use as any of the devices described below, although it is not limited thereto: a fracture fixation device such as a bone plate or a mini plate, a scaffold for a device for regenerative medicine such as an artificial bone or a skull plate, a device for treatment of a cardiovascular system such as a stent, a coil for aneurysm occlusion or a device for treating atrial septal defect, a stent for a tubular organ such as a blood vessel, a gastrointestinal tract (such as a bile

duct or an esophagus) or a trachea, and a therapeutic drug eluting device to be used such that it is placed in a tissue structure such as a bone or a blood vessel in the body. For example, to a material to be used around the bone such as a fracture fixation device, a function to accelerate bone regeneration can be imparted, and to a material to be used in the blood vessel such as a stent, a function to suppress thrombus formation or the like can be imparted.

Hereinafter, examples will be shown and an embodiment of the invention will be described in further detail. Of course, the invention is not limited to the following examples, and it is needless to say that various embodiments can be provided in the details.

### Examples

### <Example 1: Control of morphology of anodic oxide film>

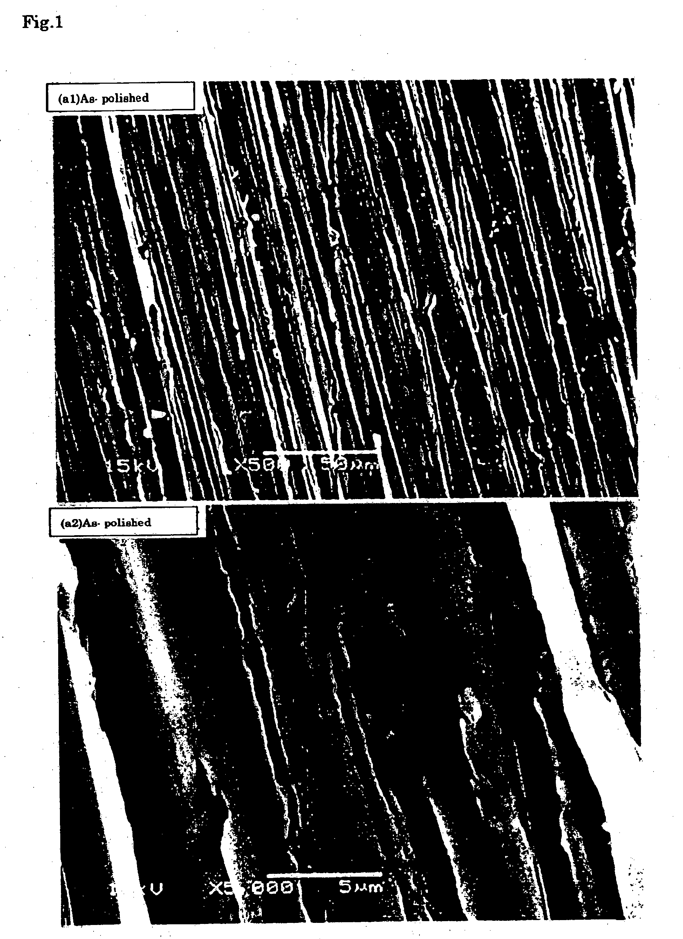

The surface of a binary magnesium alloy (a) containing 0.3 atomic % of Y was polished and the polished binary magnesium alloy (a) was immersed in 1 N NaOH solution at room temperature, and anodically oxidized under a condition of (b) 2 V, (c) 7 V, (d) 10 V, (e) 20 V, (f) 100 V or (g) 200 V, whereby an anodic oxide film was formed on the surface of the binary magnesium alloy. In Fig. 1 and Fig. 2 to Fig. 7, scanning electron microscopic (SEM) images of the as-polished surface of a binary magnesium alloy (a), and the surfaces of anodic oxide films formed under the conditions of (b) to (f) are shown, respectively. In Fig. 1 and Fig. 2 to Fig. 7, for example, (a1) shows an observation image of the binary magnesium alloy (a) with a low magnification, and (a2) shows an observation image of the binary magnesium alloy (a) with a high magnification.

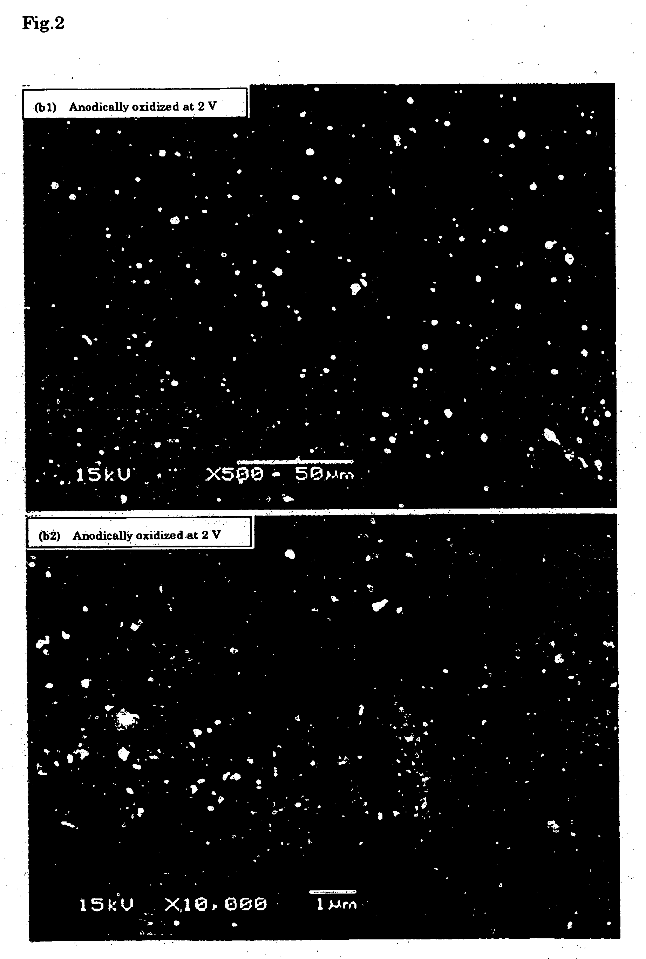

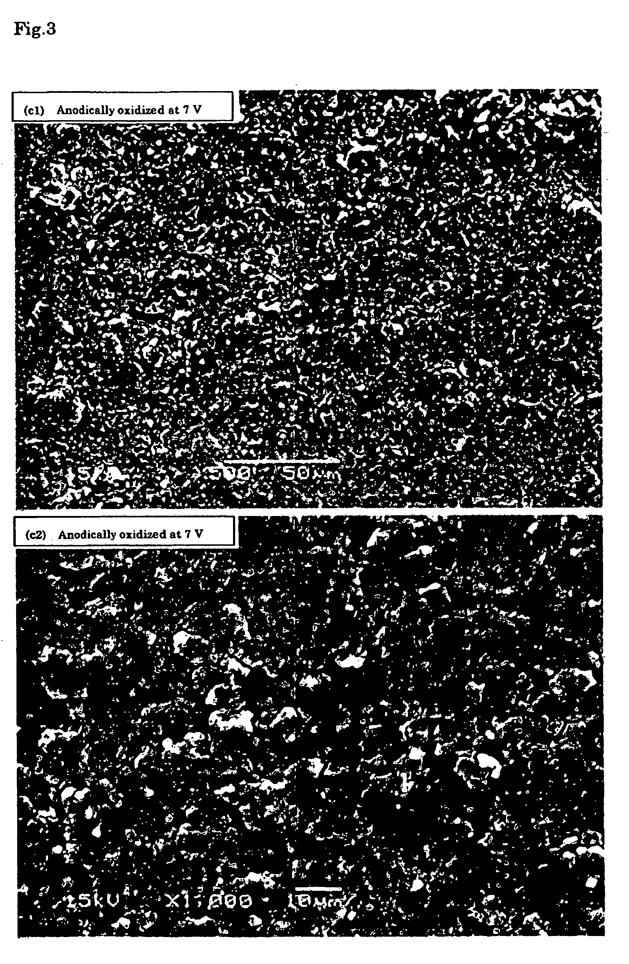

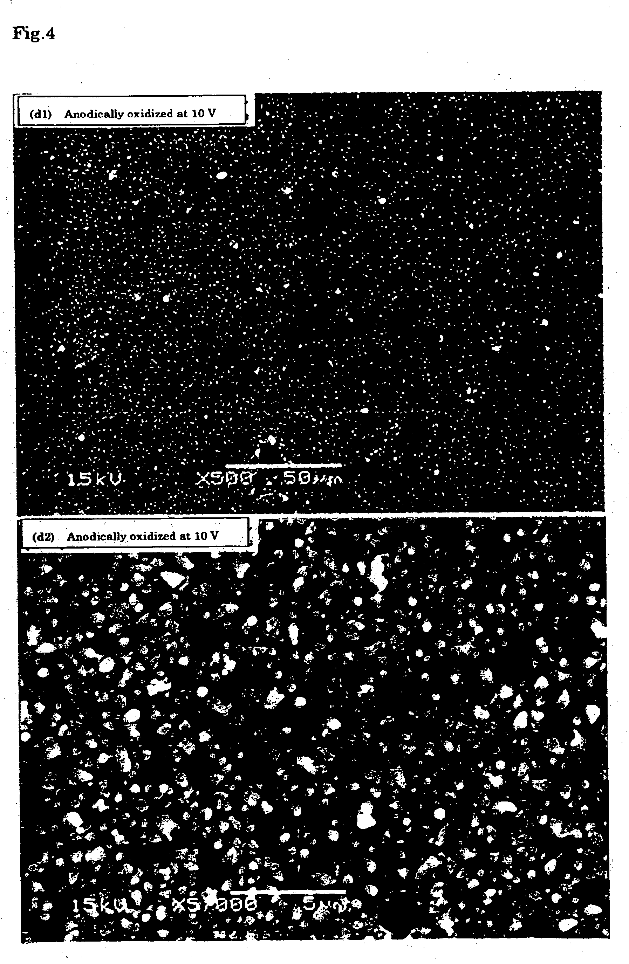

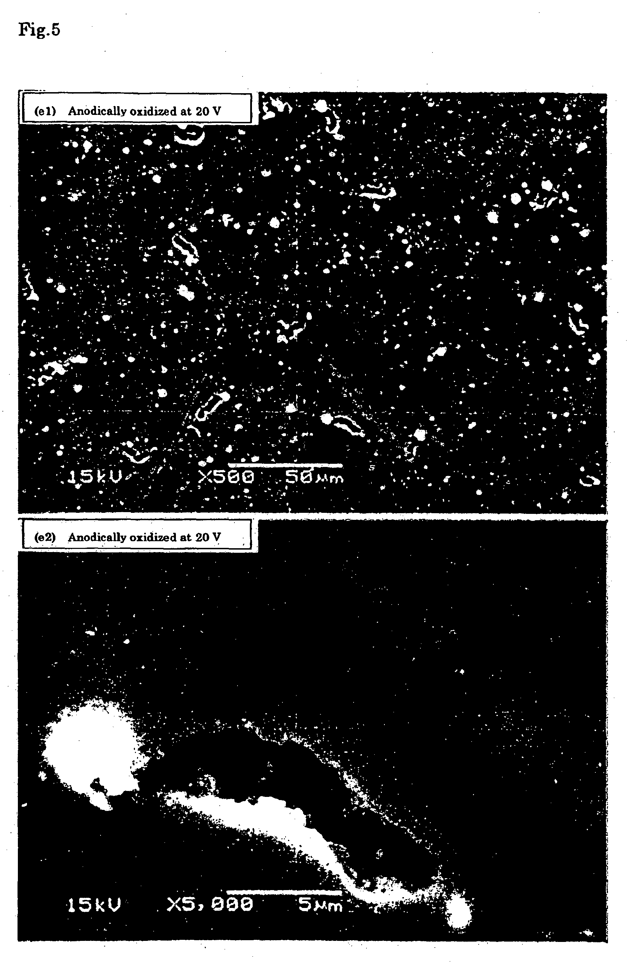

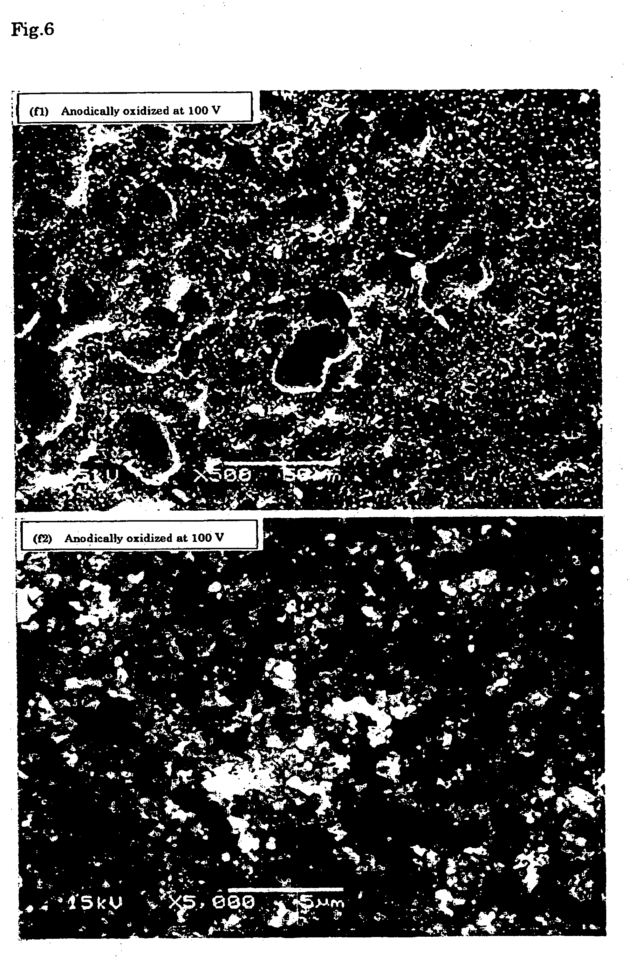

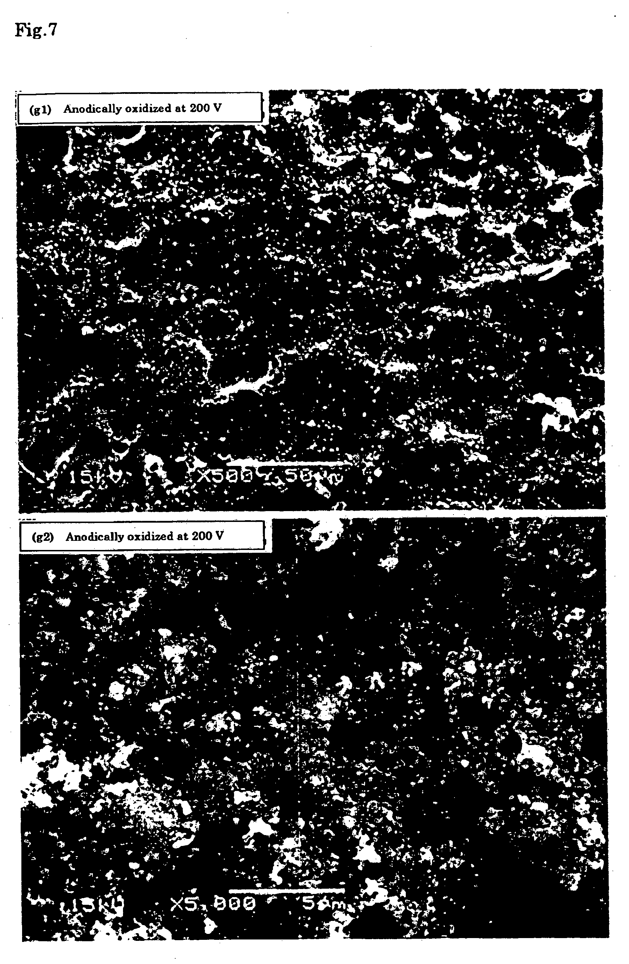

As is evident from (a1) and (a2) of Fig. 1, only polishing scars were observed on the as-polished surface. From (b1) and (b2) of Fig. 2, the surface anodically oxidized at 2 V was a very smooth surface without any unevenness or crack, however, grains with a size of 1 µm or less were scattered. From (c1) and (c2) of Fig. 3, in the film on the surface anodically oxidized at 7 V, grains with a size of 1 µm or less were formed in an aggregated manner. From (d1) and (d2) of Fig. 4, on the surface anodically oxidized at 10 V, a large number of grains with a size of 1 µm or less and pores were observed in the smooth film. From (e1) and (e2) of Fig. 5, the surface anodically oxidized at 20 V was a very smooth surface without any unevenness, however, dents with a width of 3 µm and a length of several tens micrometers were scattered. From (f1) and (f2) of Fig. 6, on the surface anodically oxidized at 100 V, concaves and convexes in a form such that a crater with a size of several tens micrometers was dug were significantly formed, and micrograins with a size of a submicron order were formed in an aggregated manner on both inside and outside of the craters. Further, also between the grains, pores with a size of a submicron order were formed. From (g1) and (g2) of Fig. 7, the surface anodically oxidized at 200 V had a similar form to that of the surface anodically oxidized at 100 V, however, the space between grains was denser than that in the case of 100 V. Accordingly, it is considered that the pore size is smaller in the case of 200 V than in the case of 100 V.

From the above results, it was revealed that by controlling the anodic oxidation voltage, the morphology of the anodic oxide film formed on the surface of a binary magnesium alloy could be controlled.

Further, it was revealed that when the voltage was set to a low voltage of around 5 V and a high voltage of 100 V or higher, the resulting film was porous, and also the porosity and pore size could be controlled by the voltage.

It becomes possible to load a drug in the pores of the thus formed film by utilizing a technique such as painting, coating, filling or immersion. Further, by controlling the porosity and pore size of the film, the surface suitable for the amount or type of the drug to be loaded and the sustained release rate of the drug can be obtained.

### <Example 2: Control of thickness of anodic oxide film>

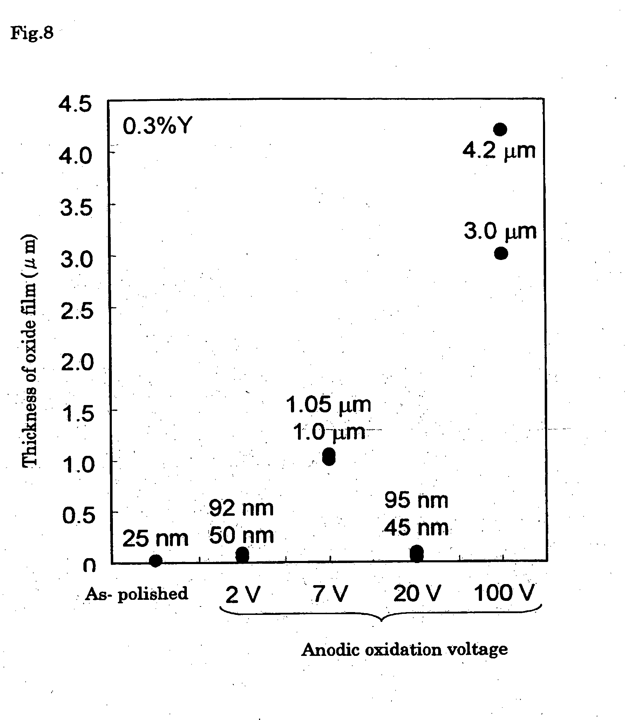

The surface of a binary magnesium alloy containing 0.3 atomic % of Y was polished and the polished binary magnesium alloy (a) was immersed in 1 N NaOH solution at room temperature, and anodically oxidized under a condition of 2 V, 7 V, 20 V or 100 V, whereby an anodic oxide film was formed on the binary magnesium alloy. In Fig. 8, the thickness of an oxide film on the as-polished surface and the surface anodically oxidized under each condition of a binary magnesium alloy is shown. The film thickness was obtained by performing a compositional analysis of each surface by Auger electron spectroscopy (AES) while performing Ar gas sputtering and calculating it from a sputtering depth at which the oxygen concentration became 50% of that of the outer-most surface.

The thickness of the oxide film on the as-polished surface and on the surface anodically oxidized at 2 V and 20 V was in the order of nanometer, however, the thickness of the oxide film on the surface anodically oxidized at 7 V and 100 V was in the order of micrometer. From these results, it was revealed that the thickness of the protective film for a magnesium based metallic material could be controlled by controlling the anodic oxidation voltage.

By controlling the thickness of the film as described above, for example, the retention period of the mechanical property of a binary magnesium alloy, the amount of a drug to be loaded on this film, and the like can be controlled.

### <Example 3: Control of composition of anodic oxide film>

The surface of a binary magnesium alloy containing 0.3 atomic % of Y was polished and the polished binary magnesium alloy was immersed in 1 N NaOH solution at room temperature, and anodically oxidized under a condition of 2 V or 20 V, whereby an anodic oxide film was formed on the binary magnesium alloy.

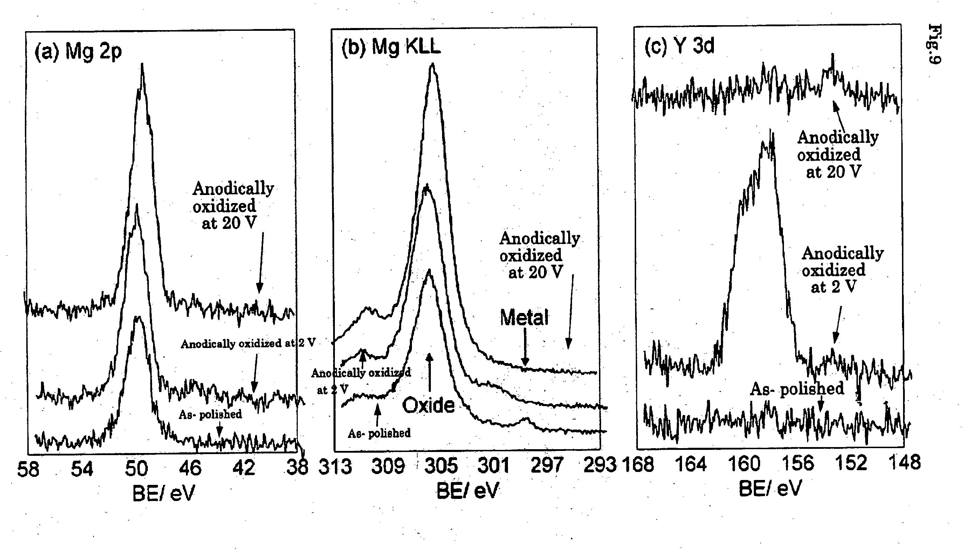

Fig. 9 shows graphs showing the X-ray photoelectron spectroscopy (XPS) spectra of the as-polished surface and the surface anodically oxidized of a binary magnesium alloy. (a) shows Mg 2p electron spectra; (b) shows Mg KLL Auger electron spectra; and (c) shows Y 3d electron spectra.

With regard to the Mg 2p electron spectra shown in Fig. 9(a), almost no difference was observed among the respective samples. With regard to the Mg KLL Auger electron spectra shown in Fig. 9(b), a broad peak originated from the metal state was observed only in the case of the as-polished surface, and it was found that the oxide film on the as-polished surface was thinnest. With regard to the Y 3d electron spectra shown in Fig. 9(c), a peak originated from Y at oxide state was observed only in the case of the surface anodically oxidized at 2V, and Y serving as the second element was concentrated in the oxide film.

From the above results, it was revealed that by controlling the anodic oxidation voltage, the composition of the film formed on the surface of a magnesium alloy could be controlled. By controlling the composition of the film, the protectiveness of the film is changed, therefore, it becomes possible to change the retention time of the strength.

### <Example 4: Anodic oxidation voltage and protectiveness of film>

A period for which the film formed on the surface of a magnesium based metallic material by anodic oxidation suppresses the degradation of the magnesium based metallic material without breaking down the film, that is, the retention time of the strength at an early stage of implantation of a device made of a magnesium based metallic material depends on the protectiveness (durability) of the film.

An increase in the anode current observed in a polarization test of a metallic material having a usual film is caused by acceleration of dissolution (ionization) of a metal due to application of an electric potential to a sample, acceleration of breakdown of the film due to a specific composition of a solution such as a chloride ion, or breakdown of the film due to intolerance of the film to an electric field applied between the substrate side and the solution side of the film. That is, with regard to an index of the protectiveness of the film, an electric potential at which a large anode current abruptly flows in a potential-current curve in a polarization test can be used as a film breakdown potential. It can be evaluated that as the film breakdown potential is higher, the protectiveness of the film is higher, that is, the period for which the film suppresses the degradation of a magnesium based metallic material is longer.

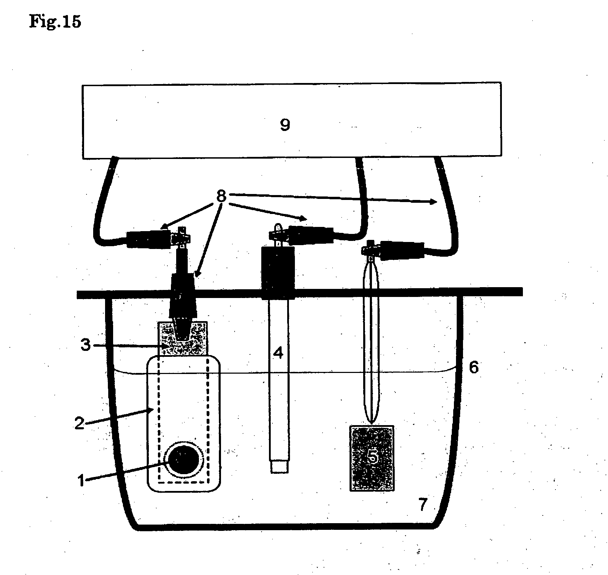

Accordingly, with respect to samples as-polished and samples anodically oxidized at a voltage of 2 V to 200 V in 1 N NaOH solution at room temperature (samples anodically oxidized) obtained by using pure magnesium with an average grain size of 1 µm (impurity concentration: 0.05 atomic % or less), binary magnesium alloys containing 0.3 atomic % of any of Y, Dy, In, Gd, Yb or Nd, and AZ31 extruded material which is a practical alloy, a polarization test was performed in an artificial body fluid having a composition shown in Table 1, and the protectiveness of the film formed by anodic oxidation was examined. Specifically, this Example 4 was performed as follows shown in Fig. 15**.**

As shown in Fig. 15, in 500 ml of an artificial body fluid (7) maintained at 37°C, a sample (1) was fixed on a stainless steel plate (3) by being covered with a silicone resin and teflon (registered trademark) tape as a fixing member (2) such that the surface of the sample (1) was vertical and exposed to the fluid. The tip of a saturated calomel electrode (SCE) was fixed as a reference electrode (4) to the vicinity of the surface of the sample (1) in a glass vessel (6). A platinum plate was fixed as a counter electrode (5) to a position facing the surface of the sample (1). These were connected to a potentiostat (9) with clips and electric wires (8). A change over time in the immersion potential of the sample (1) was monitored for 1 hour from immediately after immersion. Subsequently, the potential of the sample (1) was swept in the anodic direction from -1.8 V at a sweep rate of 1 mV/sec with respect to the SCE.

**[Table 1]**

| Table of composition of artificial body fluid and plasma (× 10⁻³ mol/l) | | |
|---|---|---|
| Composition | Artificial body fluid | Plasma |
| Na⁺ | 100 | 142 |
| K⁺ | 6 | 5 |
| Mg²⁺ | 0.8 | 1.5 |
| Ca²⁺ | 1.3 | 2.5 |
| Cl⁻ | 103 | 103 |
| HPO₄²⁻ + H₂PO₄⁻ | 0.8 | 1 |
| HCO₃⁻ | 4.2 | 27 |
| SO₄²⁻ | 0.8 | 0.5 |

This artificial body fluid is a solution containing chloride ions at a concentration equivalent to that in plasma as shown in Table 1. In general, the film of a magnesium based metallic material is liable to be broken down due to the attack by chloride ions in a solution having a near-neutral pH. A device to be implanted in the blood vessel such as a stent is exposed to blood, and a device to be implanted around soft or hard tissue such as a plate is exposed to cellular interstitial fluid. The concentration of inorganic ions in the blood and cellular interstitial fluid is equal to that in plasma, therefore, it can be considered that this Example is suitable for evaluation of the protectiveness of the film of a magnesium based metallic material. Further, the artificial body fluid in Table 1 is a solution also containing phosphate ions and calcium ions at a concentration equivalent to that in plasma, therefore, it is considered that this Example is also suitable for evaluation of an ability to precipitate calcium phosphate on the surface of the film.

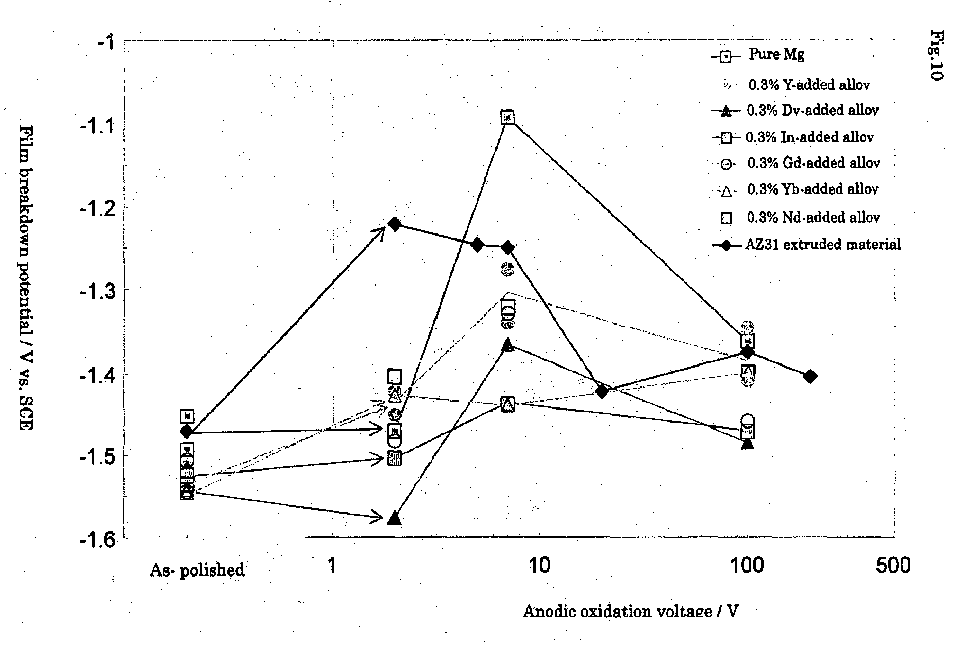

On the anodic polarization curve of the polarization curve (potential-current curve) obtained in the above-mentioned polarization test, an electric potential at which an anode current density is rapidly increased by the breakdown of the film (film breakdown potential) appeared. A relationship between this film breakdown potential and an anodic oxidation voltage is summarized in Fig. 10**.**

From Fig. 10, it was revealed that regardless of the composition of the magnesium based metallic material, the film breakdown potential was increased by anodic oxidation. On the other hand, although an increase in the film breakdown potential caused by anodic oxidation at 2 V was observed in the case of the AZ31 extruded material, in the case of pure magnesium, and binary magnesium alloys containing 0.3 atomic % of any of Y, Dy, In, Gd, Yb or Nd, an increase in the film breakdown potential caused by anodic oxidation at a voltage higher than 2 V was observed. Further, the film breakdown potential changed depending on the anode oxidation voltage.

From these results, it was shown that the degradation of a magnesium based metallic material could be controlled to be a desired retention time either short or long by changing the protectiveness of the film through anodic oxidation, and further, the period until the degradation of a magnesium based metallic material began could be controlled to be a desired period by controlling the anodic oxidation voltage.

### <Example 5: Transition of immersion potential of sample anodically oxidized>

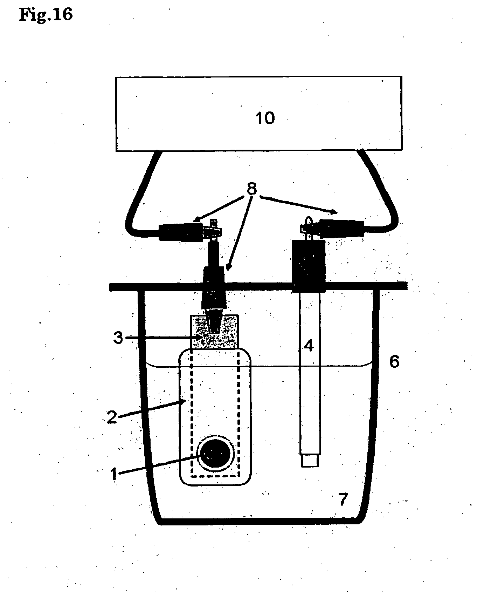

An as-polished sample of AZ31 extruded material which is a practical alloy and a sample obtained by subjecting the as-polished AZ31 extruded material to anodic oxidation at 7 V or 100 V were immersed in an artificial body fluid of a composition shown in the above Table 1, and then, an immersion potential was monitored for 2 weeks. As for the condition for immersion, 150 ml of a solution was used for a sample area of about 1 cm², and the temperature of the solution was maintained at 37°C. Specifically, as shown in Fig. 16, the immersion test was performed as follows.

As shown in Fig. 16, in 150 ml of an artificial body fluid (7) maintained at 37°C, a sample (1) was fixed on a stainless steel plate (3) by being covered with a silicone resin as a fixing member (2) such that the surface of the sample (1) was vertical and exposed to the fluid. The tip of a saturated calomel electrode (SCE) was fixed as a reference electrode (4) to the vicinity of the surface of the sample (1) in a vessel (6) made of teflon (registered trademark). These were connected to a digital X-Y recorder (10) with clips and electric wires (8). A change over time in the immersion potential of the sample (1) was monitored for 2 weeks from immediately after immersion.

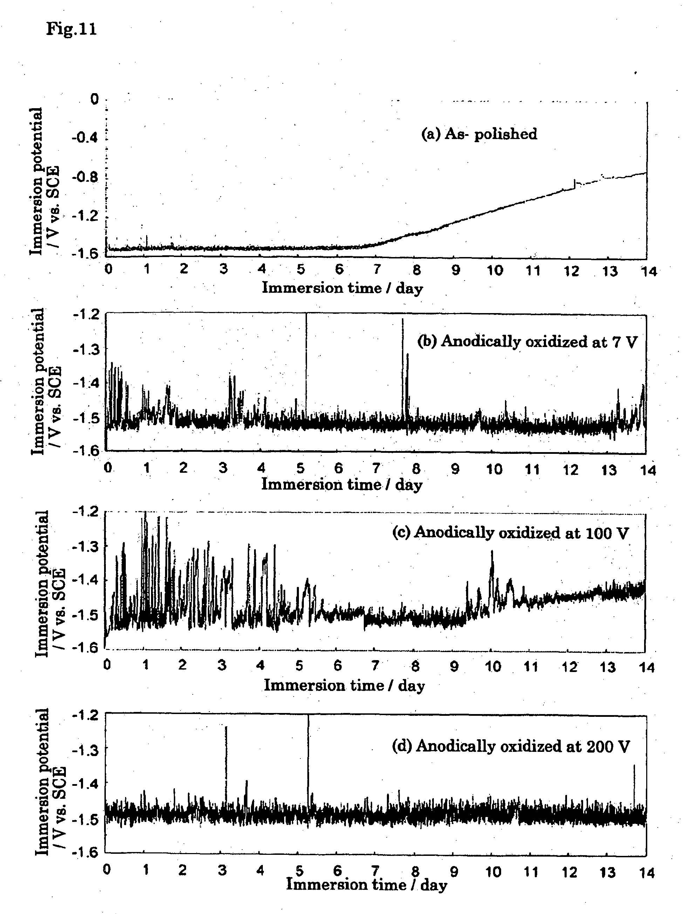

Fig. 11 shows graphs showing a transition curve of the immersion potential in the artificial body fluid for a magnesium alloy (AZ31 extruded material): (a) as-polished; and anodically oxidized (b) at 7 V; (c) at 100 V; and (d) at 200 V.

While the immersion potential at an early stage of immersion of the as-polished sample was -1.53 V (SCE), the immersion potential of the anodically oxidized sample was -1.50 V (SCE). It is generally said that as the immersion potential is higher, the protectiveness of the film on the surface of a metallic material is higher, and the results obtained this time also show that a film with a high protectiveness is formed on the surface of a magnesium based metallic material by anodic oxidation.

When the behavior of the immersion potential of the samples anodically oxidized was compared, a large spike-like potential change was frequently observed throughout the 2-week immersion period in the case of the samples anodically oxidized at 7 V or 100 V, however, in the case of the sample anodically oxidized at 200 V, a potential change was almost not observed. A change in the immersion potential is caused by local breakdown and repair of the film. From these results, it was revealed that the resistance to the local breakdown of the film was varied, that is, the protectiveness of the film was varied by the anodic oxidation voltage.

In this connection, the immersion potential for the as-polished sample and the sample anodically oxidized at 100 V began to increase at around day 7 and day 10, respectively, and the immersion potential continued to increase even at week 2 after completion of immersion. As shown in Table 2, precipitation of Ca and P was observed on the surface of each sample after completion of 2-week immersion, and a relative ratio of Ca to P of the as-polished sample and the sample anodically oxidized at 100 V was smaller than that of the samples anodically oxidized at 7 V or 200 V. From these results, it is considered that the behavior of immersion potential is varied depending on the precipitation form of calcium phosphate from the artificial body fluid.

**[Table 2]**

| AZ31 | | Surface composition before initiation of immersion (atomic %) | | | | | | |
|---|---|---|---|---|---|---|---|---|
| | | O | Mg | Al | Zn | | | |
| As-polished | | 4.5 | 92.0 | 3.2 | 0.3 | | | |

| AZ31 | | Precipitate on surface at week 2 after immersion in artificial body fluid (atomic %) | | | | | | |
|---|---|---|---|---|---|---|---|---|
| | | O | Mg | Al | Zn | Ca | P | Ca/P |
| Anodic oxidation | Non | 36.4 | 22.0 | 0.9 | 0.3 | 21.9 | 18.6 | 1.2 |
| | 7V | 24.1 | 5.3 | 0 | 0 | 44.6 | 26.0 | 1.7 |
| | 100 V | 29.8 | 41.5 | 1.4 | 0.3 | 14.3 | 12.7 | 1.1 |
| | 200 V | 36.6 | 23.8 | 1.1 | 0.2 | 23.0 | 15.3 | 1.5 |

### <Comparative example: Comparison with sample subjected to thermal oxidation in gas phase>

A film was formed by subjecting a magnesium based metallic material to thermal oxidation in the atmosphere under the same condition as that for the thermal oxidation in an oxidation atmosphere disclosed in the prior art documents (Patent document 3 and Y. A. Abdullat, S. Tsutsumi et al., Materials Science Forum (2003)), and the morphology and protectiveness of the resulting film were compared with those of the film formed by anodic oxidation of the invention. As the magnesium based metallic material on which the film was formed, a binary magnesium alloy containing 0.3 atomic % of Y was used.

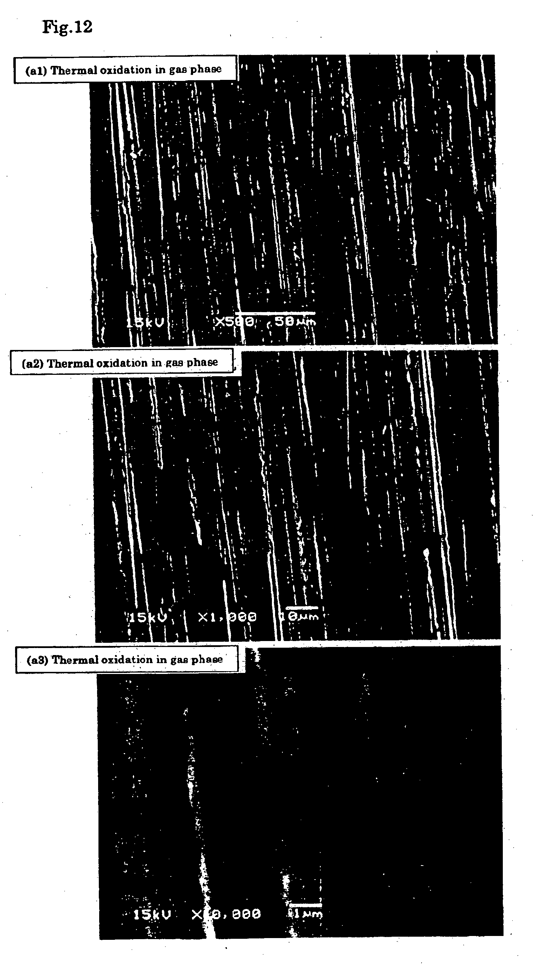

In Fig. 12, SEM images of the surface of the binary magnesium alloy containing 0.3 atomic % of Y subjected to thermal oxidation in the atmosphere are shown. On the surface of the sample, polishing scars remained, and a lot of cracks of the film were observed. Even in the observation with high magnification shown in Fig. 12(a3), pores as observed in the anodic oxide film of Example 1 were not observed. It is considered that a porous film is difficult to be formed by the thermal oxidation, and film formation through thermal oxidation is considered to be not suitable for formation of a surface for sustained release of a drug.

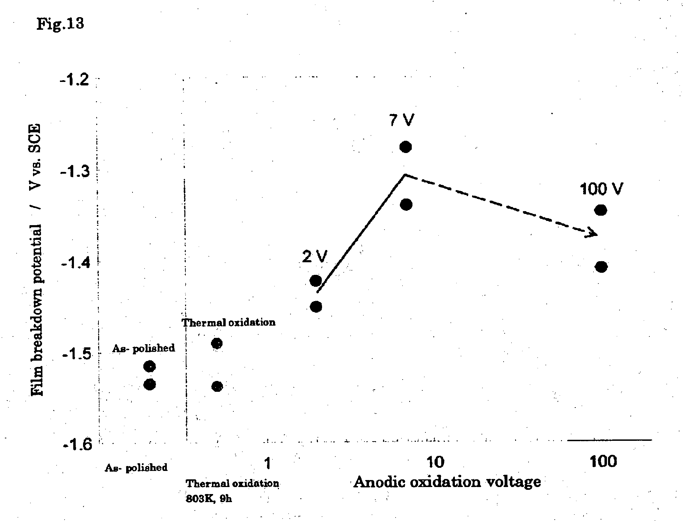

The binary magnesium alloy containing 0.3 atomic % of Y subjected to thermal oxidation in the atmosphere was immersed in an artificial body fluid, and its film breakdown potential was examined and is shown in Fig. 13. For the sake of comparison, the film breakdown potentials of the as-polished surface and alloys anodically oxidized at 2 V, 7 V or 100 V are also shown in Fig. 13. The film breakdown potential of the sample subjected to thermal oxidation was equal to that of the as-polished sample, and was lower than those of the samples anodically oxidized.

From these results, it was predicted that the protectiveness of the film formed through thermal oxidation was inferior to that of the film formed through anodic oxidation.

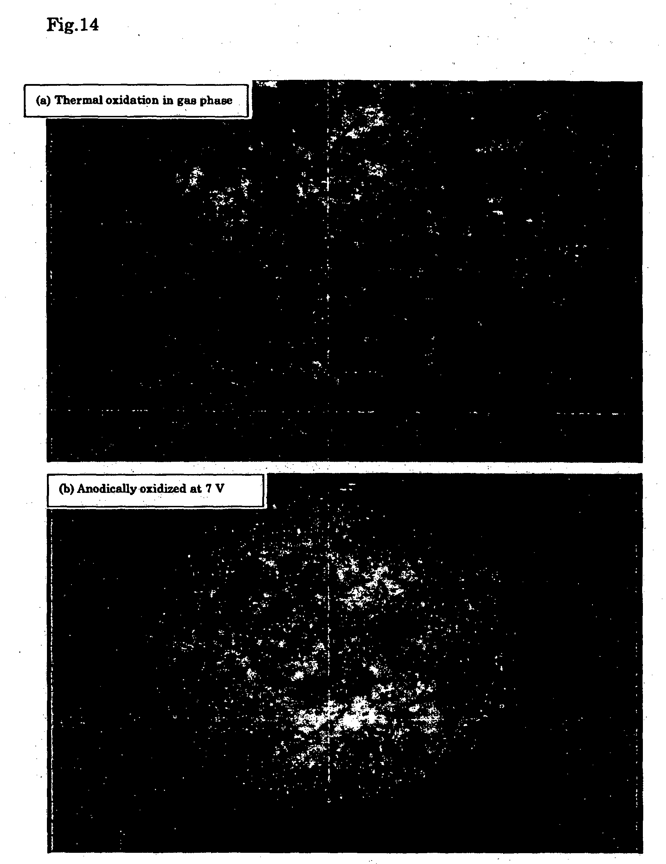

The binary magnesium alloys containing 0.3 atomic % of Y subjected to thermal oxidation in the atmosphere or anodic oxidation at 7 V were immersed in an artificial body fluid of a composition shown in the above Table 1 for 2 weeks, and the surfaces of the samples were observed with a stereomicroscope, and the observed images are shown in Fig. 14. As the respective samples, samples in the form of a disk with a diameter of 8 mm and a thickness of 2 mm were used, each of which was glued to a 316L stainless steel electrode plate with silver paste, and the 316L stainless steel electrode plate and the surface of the sample outside of the inner area of the sample with a diameter of 5 mm were covered and insulated with a PTFE tape.

When the samples were removed from the solution after they were immersed for 2 weeks, the sample subjected to thermal oxidation was completely degraded and converted into gel-like sediment. There is a possibility that the oxide film formed through thermal oxidation may be eliminated by immersion in the artificial body fluid, whereby the sample may be broken down. With regard to the sample anodically oxidized at 7 V, the original shape was maintained, however, there was a thick gel-like precipitate on the surface. This gel-like precipitate was formed uniformly on the surface of the sample subjected to anodic oxidation.

The above results of surface observation and polarization test and immersion test show that the protectiveness of the film on the surface subjected to thermal oxidation in a gas phase was lower than that of the film on the surface anodically oxidized. It was revealed that the anodic oxidation of the invention of this application is more suitable for controlling the retention time of the strength of magnesium than thermal oxidation.

### <Example 6: Protectiveness of film with respect to grain size, second composition and anodic oxidation voltage>

An experiment was conducted as to how the protectiveness of the film could be controlled by a grain size, a second composition and a voltage at the time of anodic oxidation. The experimental results are shown in Table 3.

The preparation method of samples, anodic oxidation method and measurement method of the protectiveness of the film were the same as in this Example 4.

**[Table 3]**

| Film breakdown potential* obtained by polarization test (artificial body fluid) (/V vs. SCE) | | | | | | |
|---|---|---|---|---|---|---|
| Magnesium based metallic material | | Average grain size of grains (µm) | As-polished | Anodic oxidation | | |
| | | | | 2 V | 7 V | 100 V |
| a | Pure Mg | 1 | -1.56 | -1.45 | -1.37 | -1.49 |
| b | Pure Mg | 5 | -1.55 | -0.37 | -0.85 | -0.97 |
| c | Pure Mg | 50 | -1.48 | -0.07 | -0.37 | 1.38 |
| d | Pure Mg | 100 | -1.47 | -1.47 | -1.09 | 0.50 |
| e | Pure Mg | 200 | -1.51 | 0.09 | -0.55 | -1.43 |
| f | 0.3% Li-added alloy | 1 | -1.37 | -0.52 | -1.58 | -1.43 |
| g | 0.3% Ca-added alloy | 1 | -1.01 | -1.45 | -0.28 | -0.48 |
| h | 0.3% Al-added alloy | 1 | -1.50 | -0.61 | -0.25 | -1.33 |
| i | 0.3% In-added alloy | 1 | -1.60 | -1.06 | -1.44 | -1.47 |

| | | | | | | |
|---|---|---|---|---|---|---|
| * In the case where there were two or more data, an average value of the data was used. | | | | | | |

From Table 3, even pure magnesium showed a different film breakdown potential according to the difference in the grain size even in the case where anodic oxidation was performed at the same voltage. From these results, it was shown that by combining the grain size of a magnesium based metallic material with the condition of anodic oxidation, the degradation of a magnesium based metallic material could be controlled to a desired retention time.

Further, it was revealed that there are elements which give a higher film breakdown potential through anodic oxidation and elements which give an equal or lower film breakdown potential according to the type of element to be added as the second composition. From these results, it was shown that by combining the type of element to be added with the condition of anodic oxidation, the degradation of a magnesium based metallic material could be controlled to a desired retention time, either short or long.

## Claims

1. A method of controlling a degradation time of a biodegradable device for medical use, which is a method of controlling a degradation time in vivo of
a biodegradable device for medical use containing a biodegradable magnesium material or magnesium alloy material for medical use which is degraded and absorbed in vivo as a substrate, **characterized by** controlling the degradation time in vivo by forming a film containing magnesium oxide and/or magnesium hydroxide by anodic oxidation on the surface of the substrate and controlling morphology of the film thereby controlling the porosity or pore size of the film, wherein the anodic oxidation is effected by applying charge using magnesium or a magnesium alloy as an anode in an electrolyte containing one or more compositions selected from the group consisting of a salt or a hydroxide of sodium, potassium, aluminium or calcium, a salt of phosphoric acid, silicic acid, aluminic acid, boric acid, oxalic acid, acetic acid or tartaric acid, a fluoride and ethylene glycol.

2. The method of controlling a degradation time of a biodegradable device for medical use according to claim 1, **characterized in that** time, a voltage and a current when an electric charge is applied are controlled in order to vary a desired morphology of the film to the surface of magnesium or a magnesium alloy.

## Patentansprüche

1. Verfahren zum Kontrollieren der Degradationszeit einer biologischen abbaubaren Vorrichtung zur medizinischen Verwendung, welches ein Verfahren zum Kontrollieren einer Abbauzeit in vivo einer biologisch abbaubaren Vorrichtung zur medizinischen Verwendung enthaltend ein biologisch abbaubares Magnesiummaterial oder Magnesiumlegierungsmaterial zur medizinischen Verwendung ist, welches in vivo als ein Substrat abgebaut und absorbiert wird, **gekennzeichnet durch** Kontrollieren der Abbauzeit in vivo **durch** Bilden eines Films enthaltend Magnesiumoxid und/oder Magnesiumhydroxid **durch** anodische Oxidation auf der Substratoberfläche und Kontrollieren der Morphologie des Films, wobei die Porosität oder Porengröße des Films kontrolliert wird, wobei die anodische Oxidation **durch** Anlegen einer Ladung unter Verwendung von Magnesium oder einer Magnesiumlegierung als eine Anode in einem Elektrolyten ausgeführt wird, der ein oder mehrere Zusammensetzungen enthält, die ausgewählt sind aus der Gruppe bestehend aus einem Salz oder einem Hydroxid von Natrium, Kalium, Aluminium oder Calcium, einem Salz der Phosphorsäure, Kieselsäure, Aluminiumsäure, Borsäure, Oxalsäure, Essigsäure oder Weinsäure, Fluorid und Ethylenglykol.

2. Verfahren zum Kontrollieren der Degradationszeit eines biologisch abbaubaren Vorrichtung zur medizinischen Verwendung nach Anspruch 1, **dadurch gekennzeichnet, dass** die Zeit, eine Spannung und ein Strom kontrolliert werden, wenn eine elektrische Ladung angelegt wird, um eine gewünschte Morphologie des Films auf der Oberfläche von Magnesium oder einer Magnesiumlegierung zu variieren.

## Revendications

1. Procédé de commande d'un temps de dégradation d'un dispositif biodégradable à usage médical, qui est un procédé de commande d'un temps de dégradation in vivo d'un dispositif biodégradable à usage médical contenant une matière de magnésium ou une matière d'alliage de magnésium biodégradable à usage médical qui est dégradée et absorbée in vivo comme un substrat, **caractérisé par** la commande du temps de dégradation in vivo en formant un film contenant de l'oxyde de magnésium et/ou de l'hydroxyde de magnésium par oxydation anodique sur la surface du substrat et en maîtrisant la morphologie du film commandant ainsi la porosité ou la taille de pore du film, dans lequel l'oxydation anodique est effectuée en appliquant une charge en utilisant du magnésium ou un alliage de magnésium comme anode dans un électrolyte contenant une ou plusieurs compositions sélectionnées à partir du groupe consistant en un sel ou un hydroxyde de sodium, potassium, aluminium ou calcium, un sel d'acide phosphorique, acide silicique, acide aluminique, acide borique, acide oxalique, acide acétique ou acide tartrique, un fluorure et un éthylène glycol.

2. Procédé de commande d'un temps de dégradation d'un dispositif biodégradable à usage médical selon la revendication 1, **caractérisé en ce que** le temps, une tension et un courant quand une charge électrique est appliquée sont commandés afin de modifier une morphologie souhaitée du film sur la surface de magnésium ou d'un alliage de magnésium.
